# EUROPEAN PATENT SPECIFICATION

(11) **EP 3 153 187 B1**
(45) Date of publication and mention of the grant of the patent: **14.02.2018**
(21) Application number: 16192013.7
(22) Date of filing: 03.10.2016
(51) Int. Cl.: A61L 27/18

(54) **A TISSUE SCAFFOLD PRODUCTION METHOD**
GEWEBEGERÜSTHERSTELLUNGSVERFAHREN
PROCÉDÉ DE PRODUCTION D'UN ÉCHAFAUDAGE DE TISSU

(30) Priority: 09.10.2015 TR 201512556
(43) Date of publication of application: 12.04.2017
(73) Proprietor: Yeditepe Universitesi, 34755 Istanbul (TR)
(72) Inventor: CAN, ERDE, 34755 ISTANBUL (TR); TORUN KOSE, GAMZE, 34755 ISTANBUL (TR); CEMALI, GORKEM, 34755 ISTANBUL (TR)
(74) Representative: Dericioglu Kurt, Ekin

(56) References cited:
- US-A- 6 153 664
- US-A1- 2008 206 308
- US-A1- 2013 071 464

## Description

### Field of the Invention

The present invention relates to a production method of a biodegradable and biocompatible tissue scaffold which is designed to support cell amplification by being applied to the tissue during tissue regeneration after defect of a cartilage and/or bone tissue.

### Background of the Invention

Various biodegradable and biocompatible polymer tissue scaffolds have been used for cell regeneration in cartilage and bone tissue engineering. The main substances that have been used in those studies are hydroxyapatite, polylactic acid (PLLA) and polycaprolactone (PCL).

There are different polymer compositions granted with patents for cartilage and/or bone tissue scaffolds. The polymers used in those patents are mainly PLLA, PCL and PLLA-PCL.

Additionally, use of polypropylene fumarate (PPF) and vinyl pyrrolidone (VP) together for construction of scaffold is known. PPF is an unsaturated polyester. Radical polymerization of PPF in the presence of VP gives a cross-linked, biocompatible and biodegradable thermoset polymer. A porous form is provided to these polymers by means of techniques such as salt leaching. In the recent years, various studies have been conducted on compositions of PPF with VP or different comonomers and on composites thereof with inorganic additives. Positive results have been obtained in biocompatibility, biodegradability and mechanical properties regarding use of these materials as bone tissue scaffold and these studies have been published. ¹
¹ - He, S., Yaszemski, M.J., Yasko, A.W., Engel, P.S., Mikos, A.G. 2000. "Injectable biodegradable polymer composites based on poly(propylene fumarate) crosslinked with poly(ethylene glycol)-dimethacrylate", Biomaterials, 21, 2389-2394.
   - Kempen, D.H., Lu, L., Zhu, X., Kim, C., Jabbari, E., Dhert, W.J., Currier, B.L., Yaszemski, M.J. 2004. "Development of biodegradable poly(propylene fumarate)/poly(lactic-co-glycolic acid) blend microspheres. I. preparation and characterization", Journal of Biomedical Materials Research. Part A, 70, 283-292.
   - Lee, K., Wang, S., Yaszemski, M.J., Lu, L. 2008. "Physical properties and cellular responses to crosslinkable poly(propylene fumarate)/hydroxyapatite nanocomposites", Biomaterials, 29, 2839-2848.
   - Lee, J.W., Lan, P.X., Kim, B., Lim, G., Cho, D.W. 2008. "Fabrication and characteristic analysis of a poly(propylene fumarate) scaffold using micro-stereolithography technology", Journal of biomedical materials research. Part B, Applied biomaterials, 87, 1-9.
   - Peter, S.J., Kim,P., Yasko, S.W., Yaszemski, M.J., Mikos, A.G. 1999. "Crosslinking characteristics of an injectable poly(propylene fumarate)/β-tricalcium phosphate paste and mechanical properties of the crosslinked composite for use as a biodegradable bone cement", Journal of Biomedical Materials Research, 44, 314-321.
   - Timmer, M.D., Ambrose, C.G., Mikos, A.G. 2003. "Evaluation of thermal- and photo-crosslinked biodegradable poly(propylene fumatate)-based networks", Journal of Biomedical Materials Research. Part A, 66, 811-818.

US 6 153 664 A discloses bioerodible polymeric semi-interpenetrating networks a first bioerodible polymer capable of producing acidic products upon hydrolytic degradation and a second bioerodible polymer, which via crosslinking, provides a biopolymeric scaffolding. The second bioerodible polymer comprises polypropylene fumarate (PPF) which is cross-linked, desirably by a vinyl monomer such as vinyl pyrrolidone (VP) to form the biopolymeric scaffolding which provides dimensional and geometric stability. The scaffold can be used for bone repair. However the required improvements that need to be made in the classic applications in the state of the art have not been provided yet. Difficulty of synchronizing biodegradation rate to tissue healing rate, receiving undesirable immune responses in the event of accumulation of biodegradation products, difficulty of the body in buffering the pH changes produced by the biodegradation products, inability to provide sufficient induction for tissue regeneration can be mentioned as the main problems of the state of the art.

### Summary of the Invention

An objective of the present invention is to provide a tissue scaffold production method wherein a biomaterial for use in regeneration of bone and cartilage tissues is obtained.

Another objective of the present invention is to provide a production method for a porous or non-porous tissue scaffold having a biodegradability and biocompatibility higher than the tissue scaffolds in the state of the art.

A further objective of the present invention is to provide a tissue scaffold production method wherein biostructures which can be applied to different bone tissues are obtained by changing the comonomer and/or initiator ratios.

### Detailed Description of the Invention

"A tissue scaffold production method" developed to fulfill the objective of the present invention is illustrated in the accompanying figures, in which;
- Figure 1.: is the flow chart of the method of the present invention.
- Figure 2.: is the FT-IR spectrum of the PPF pre-polymer synthesized in the method of the present invention.
- Figure 3.: is the table wherein the FT-IR peak assignments of functional groups of the PPF pre-polymer synthesized in the method of the present invention are shown.
- Figure 4.: is the H¹-NMR spectrum of the PPF pre-polymer synthesized in the method of the present invention.
- Figure 5.: shows the results of the analysis of equilibrium water content for different compositions of the PPF-VPA and PPF-VPES polymers.
- Figure 6.: is the pH change graphic of PPF-VPES polymers at different compositions in PBS buffer solution at 37°C within a period of 80 days.
- Figure 7.: is the pH change graphic of PPF-VPA polymers at different compositions in PBS buffer solution at 37°C within a period of 80 days.
- Figure 8.: is the pH change graphic of PPF-VPA and PPF-VPES polymers containing 60% by weight of PPF in PBS buffer solution at 37°C within a period of 80 days.
- Figure 9.: is the SEM image of PPF-VPA porous polymer containing 60% by weight of PPF produced by the method of the invention at 50X magnification.
- Figure 10.: is the SEM image of PPF-VPA porous polymer containing 60% by weight of PPF produced by the method of the invention at 1500X magnification.
- Figure 11.: is the SEM image of PPF-VPA porous polymer containing 60% by weight of PPF produced by the method of the invention at 3000X magnification.
- Figure 12.: is the SEM image of PPF-VPES non-porous polymer containing 60% by weight of PPF produced by the method of the invention at 500X magnification.
- Figure 13.: is the SEM image of PPF-VPES nonporous polymer containing 60% by weight of PPF produced by the method of the invention at 3000X magnification.
- Figure 14.: is a comparative graphic showing daily cell growth profiles of the PPF-VP, PPF-VPA and PPF-VPES polymers containing 60% by weight of PPF and those of the control sample by means of MTS assay.

The tissue scaffold production method (100) of the present invention comprises the steps of
- synthesizing polypropylene fumarate (PPF) pre-polymer (101),
- mixing PPF with vinyl phosphonic acid (VPA) or vinyl phosphonic acid ester (VPES) (102),
- curing the mixture by heating it in the presence of a radical initiator (103),
- obtaining the porous or nonporous biopolymer that will be used as tissue scaffold (104).

In the tissue scaffold production method (100) of the present invention, firstly synthesis of PPF pre-polymer is performed via polycondensation reaction (101). For this purpose, fumaric acid and propylene glycol are subjected to polycondensation reaction in the presence of hydroquinone radical inhibitor and p-toluene sulfonic acid catalyst; and since the reaction takes place in excess of propylene glycol, PPF pre-polymer is obtained in hydroxyl-terminated form. In the preferred embodiment of the invention, 0.1-0.3% by weight of hydroquinone and 1-2% by weight of p-toluene sulfonic acid are used.

The polycondensation reaction taking place in step 101 is shown schematically below.

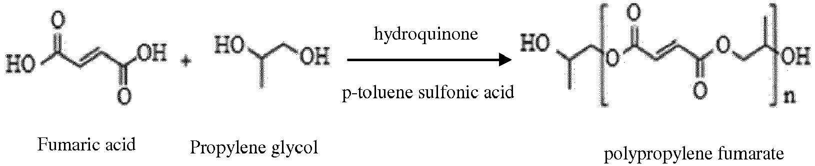

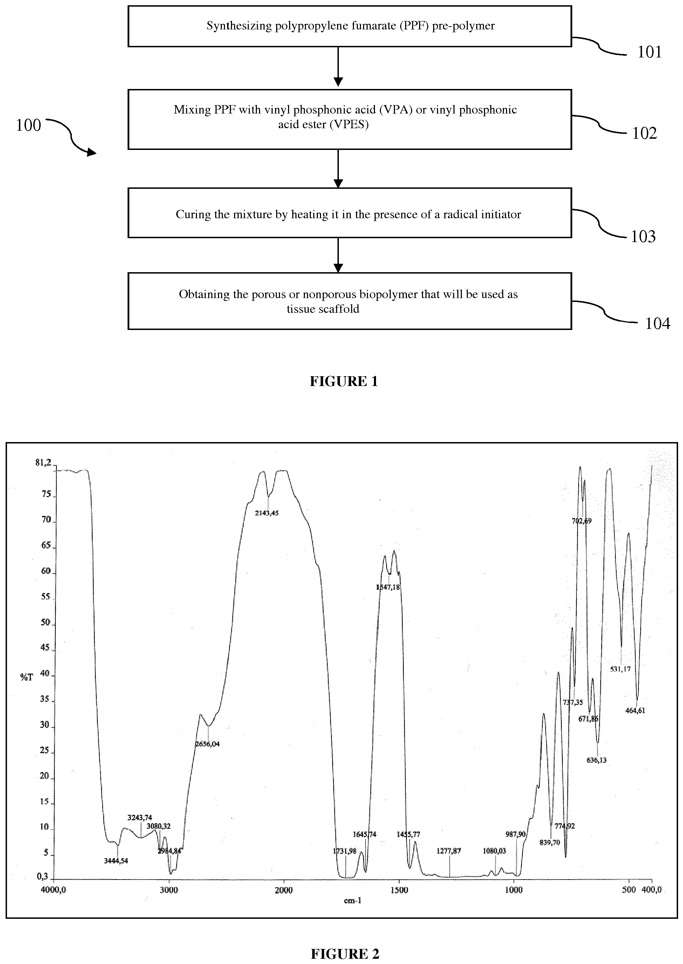

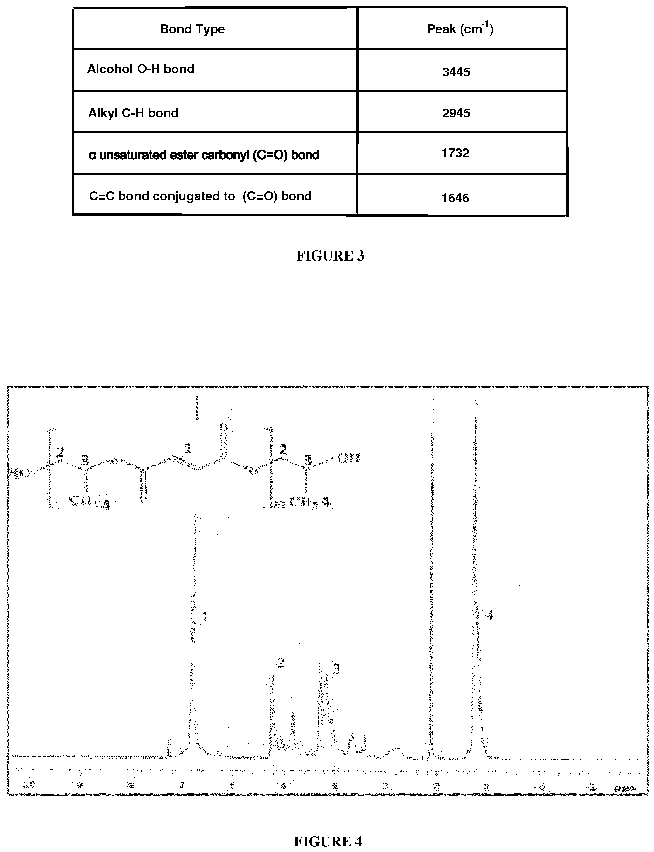

FT-IR spectrum of the synthesized PPF pre-polymer is shown in Figure 2; and the functional groups on the PPF structure to which the characteristic peaks observed in this spectrum belong are shown in Figure 3.

¹H-NMR spectrum of PPF pre-polymer is given in Figure 4 and the peaks of different protons in the PPF structure can be seen in this figure.

In one embodiment of the invention, PPF pre-polymer was mixed with vinyl phosphonic acid (VPA) comonomer in the presence of benzoyl peroxide (102). The amount of benzoyl peroxide was preferably 2-3% by weight. Amount of PPF in the mixture is 10-90% by weight.

VPA structure is as follows:

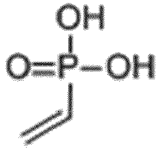

In the preferred embodiment of the invention, PPF pre-polymer was mixed with vinyl phosphonic acid ester (VPES) comonomer in the presence of benzoyl peroxide (102). The amount of benzoyl peroxide was preferably 2-3% by weight. Amount of PPF in the mixture was 10-90% by weight.
VPES structure is as follows:

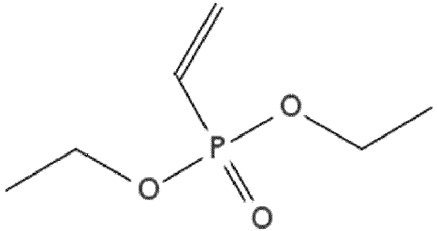

The prepared mixture was cured at different temperatures and for different periods of time in a temperature-controlled oven (103). In the preferred embodiment of the invention, the mixture was cured in the presence of benzoyl peroxide approximately at 60°C for 2 hours, at 85°C for 2 hours and at 100°C for 5 hours, respectively.

After the curing process (103), the biopolymer to be used as tissue scaffold was obtained in the form of a porous PPF-VPA mixture or nonporous PPF-VPES mixture (104).

PPF-VPA and PPF-VPES mixtures having different ratios were prepared in order to realize the characterizations of the tissue scaffolds produced with the method of the present invention (100) and several analyses were conducted on them.

In order to be able to make a comparison with the state of the art, polypropylene fumarate and vinyl pyrrolidone (PPF-VP) mixture was also prepared in the scope of the method of the invention, and a tissue scaffold having this polymeric structure was obtained.

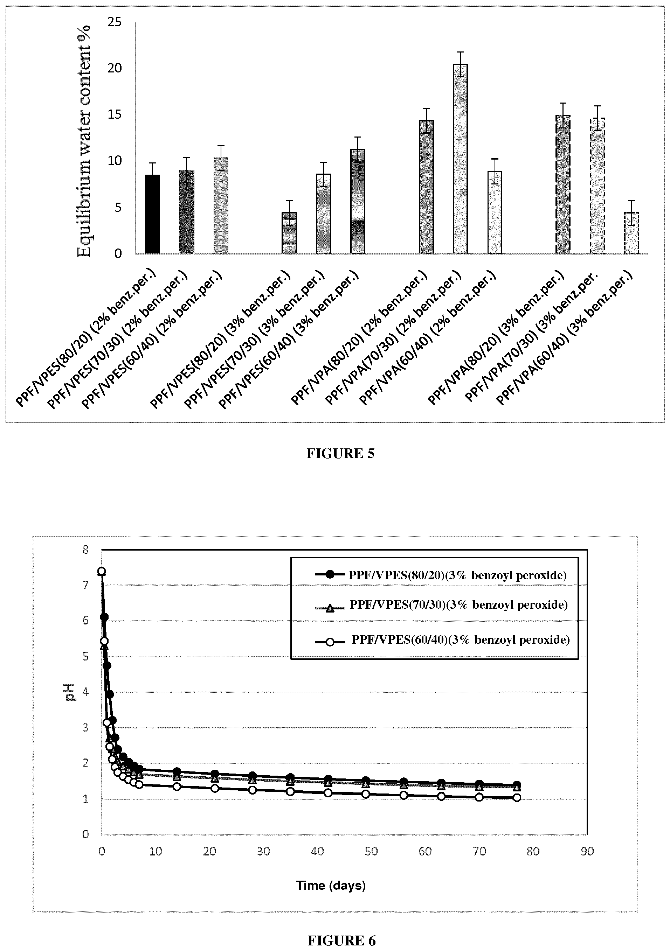

Formation (curing) of the obtained PPF-VP, PPF-VPA and PPF-VPES polymers was monitored by FT-IR spectroscopy. Equilibrium water content analyses for different compositions of the PPF-VPA and PPF-VPES polymers were performed. Data of the mentioned analyses are shown in Figure 5.

Thermomechanical properties and mechanical properties of PPF-VPA and PPF-VPES polymers such as glass transition points and compression moduli were determined by DMA (dynamic mechanical analysis) tests.

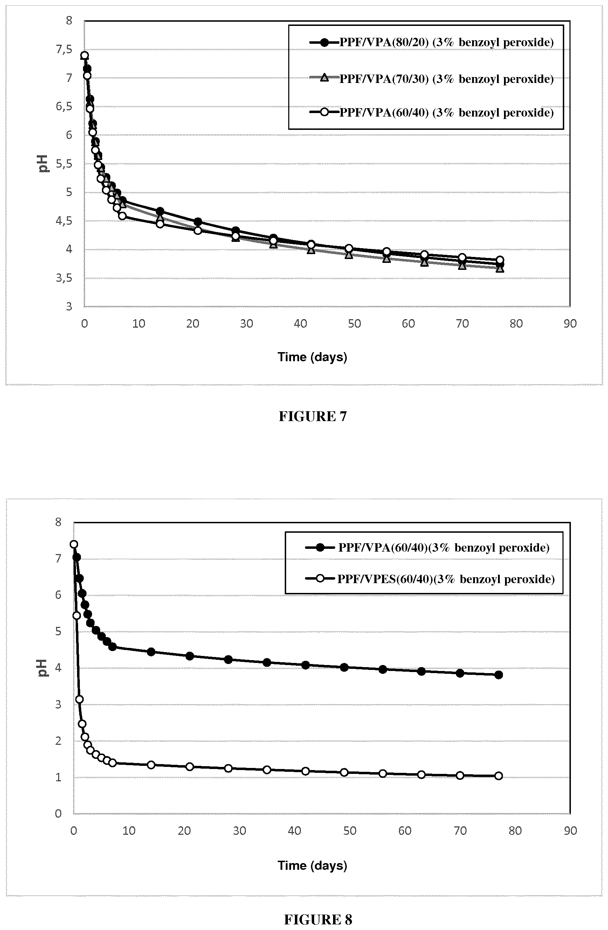

Studies on biodegradation of polymers were conducted in PBS buffer solutions at 37°C. Since as a result of degradation of PPF polymer; fumaric acid and, in the degradation of PPF-VPA based polymers, additionally polyvinyl phosphonic acid (PVPA) and/or vinyl phosphonic acid (VPA) are released; biodegradation studies were performed by pH measurement and the biodegradation rates for different compositions were compared in Figure 6 to Figure 8.

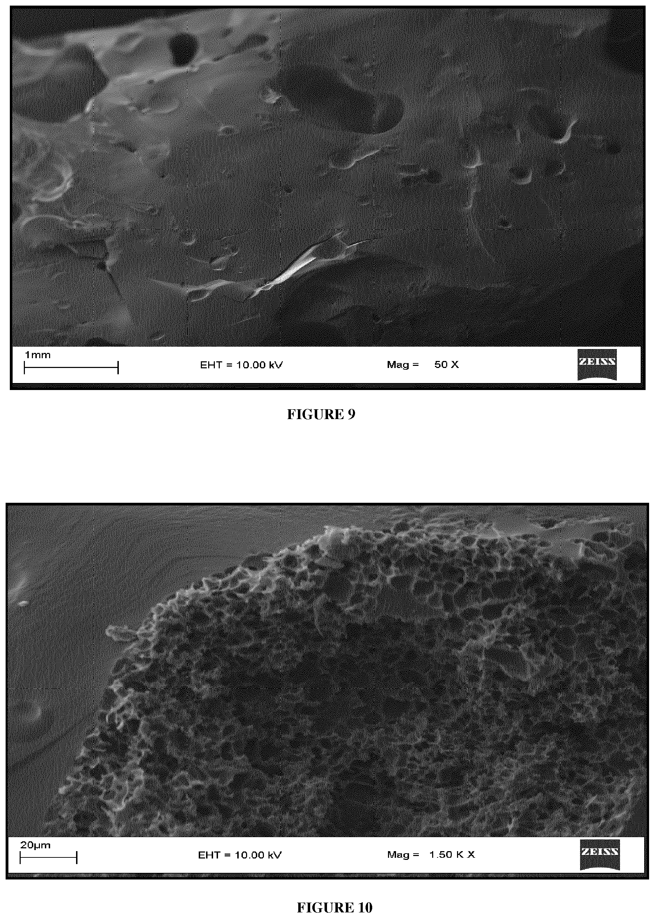

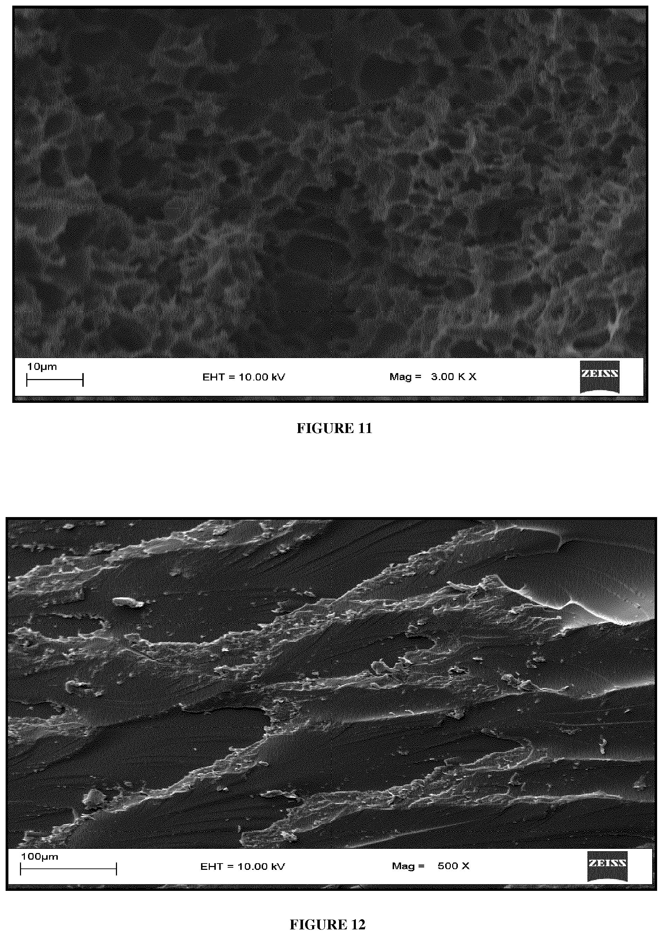

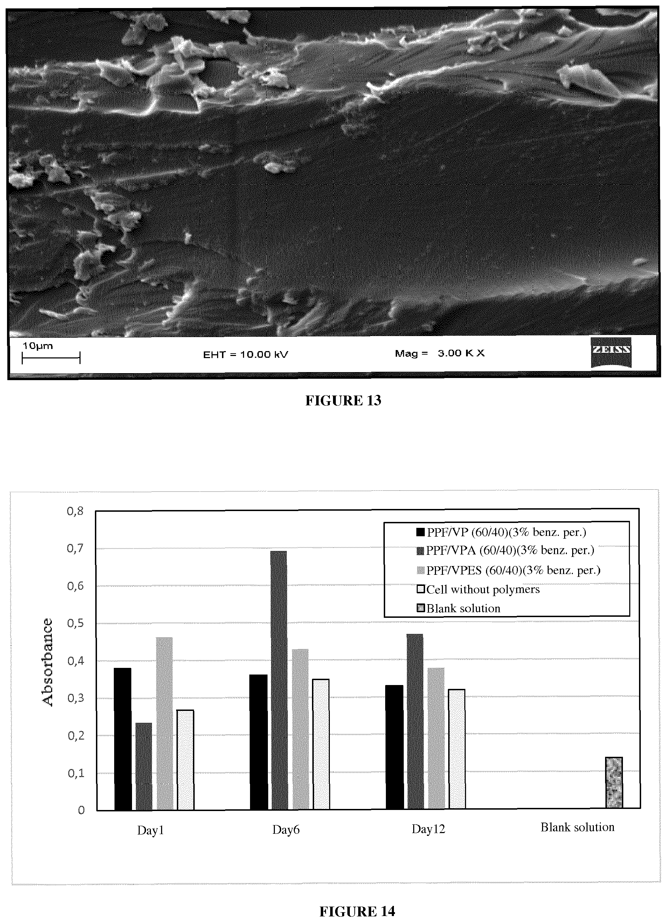

SEM images of the surface of the PPF-VPA polymer comprising 60% by weight of PPF and 40% by weight of VPA cured by 3% benzoyl peroxide (BP) are given in Figure 9 to 11 to exhibit the pore sizes of the PPF-VPA polymers. The large pores seen in Figure 9 were not dispersed homogenously on the polymer surface. In Figure 10 and 11, images taken by being focused on a specific region on the material surface show that the sizes of the pores on this inner structure were in the range of 1-5 µm. The said porous structure was considered to be formed in connection with evaporation of the approximately 10% water in the VPA monomer during the curing process. Similar porous structure was not observed in different compositions of the PPF/VPES polymers. In Figures 12 and 13, SEM images of the surface of the PPF-VPES polymer containing 60% by weight of PPF showed the nonporous surface structure of these polymers.

MTS assays were performed on PPF-VP, PPF-VPA and PPF-VPES polymers containing 60% by weight of PPF in order to observe cell proliferation. MTS assay is a colorimetric method and is used for determining the number of cells that can attach and proliferate on/in the materials. If the cells are dead, they do not convert the MTS solution to formazan. The amount of formazan that is formed is directly proportional to the number of viable cells on the material surface.

In MTS assay, absorption values read at 490 nm were recorded. For each sample, MTS assays were performed, average absorption values were taken, and the comparative graphic is given in Figure 14.

Figure 14 shows the absorbances of cell containing PPF-VP, PPF-VPA and PPF-VPES polymers in day 1, 6 and 12 and for comparison purposes those of the control sample (cell without polymers) and the blank solution on the same days. As expected, PPF-VPA and PPF-VPES polymers showed a much higher absorbance value than the PPF-VP polymers.

As a result of the analyses that were performed, it was observed that cell attachment and growth in PPF-VPA and PPF-VPES compositions were higher than the control sample, and especially after day 6, the highest cell growth occurred in the PPF-VPA composition containing 60% by weight of PPF and 40% by weight of VPA. For this reason, the most preferred embodiment of the invention was realized by the steps of mixing 60% weight of PPF with 40% by weight of VPA (102) and obtaining PPF-VPA biopolymer containing 60% by weight of PPF (104).

In the tissue scaffold production method (100) of the present invention, by changing the PPF and comonomer ratios, it is possible to produce biopolymers that can be applied to different bone and cartilage defects.

## Claims

1. A tissue scaffold production method (100), which is designed to support cell amplification by being applied to the tissue during tissue regeneration after defect of a cartilage and/or bone tissue, **characterized by** the steps of
- synthesizing polypropylene fumarate (PPF) pre-polymer (101),
- mixing PPF with vinyl phosphonic acid (VPA) or vinyl phosphonic acid ester (VPES) (102),
- curing the mixture by heating it in the presence of a radical initiator (103),
- obtaining the porous or nonporous biopolymer that will be used as tissue scaffold (104).

2. Tissue scaffold production method (100) according to Claim 1, **characterized by** the step of synthesizing PPF pre-polymer (101) wherein fumaric acid and propylene glycol are subjected to polycondensation reaction in the presence of hydroquinone radical inhibitor and p-toluene sulfonic acid catalyst; and since the reaction takes place in excess of propylene glycol, PPF pre-polymer is obtained in hydroxyl-terminated form.

3. Tissue scaffold production method (100) according to Claim 2, **characterized by** the step of synthesizing PPF pre-polymer (101) wherein 0.1-0.3% by weight of hydroquinone and 1-2% by weight of p-toluene sulfonic acid are used.

4. Tissue scaffold production method (100) according to Claim 3, **characterized by** the step of mixing PPF pre-polymer with vinyl phosphonic acid (VPA) comonomer (102) in the presence of 2-3% by weight of benzoyl peroxide.

5. Tissue scaffold production method (100) according to Claim 3, **characterized by** the step of mixing PPF pre-polymer with vinyl phosphonic acid ester (VPES) comonomer (102) in the presence of 2-3% by weight of benzoyl peroxide.

6. Tissue scaffold production method (100) according to Claim 4 or 5, **characterized by** the step of mixing PPF with vinyl phosphonic acid (VPA) or vinyl phosphonic acid ester (VPES) (102) wherein the PPF amount in the mixture is 10-90% by weight.

7. Tissue scaffold production method (100) according to Claim 6, **characterized by** the step of curing the prepared mixture (103) in a temperature-controlled oven in the presence of benzoyl peroxide at 60°C for 2 hours, at 85°C for 2 hours and at 100°C for 5 hours, respectively.

8. Tissue scaffold production method (100) according to Claim 7, **characterized in that,** after the curing process (103), the biopolymer to be used as tissue scaffold is obtained in the form of a porous PPF-VPA mixture or nonporous PPF-VPES mixture (104).

9. A porous, biodegradable and biocompatible tissue scaffold comprised of PPF-VPA mixture containing 60% by weight of PPF, which is produced by the method according to Claim 8.

## Patentansprüche

1. Ein Produktionsmethode für Gewebegerüst (100), die entworfen ist für die Unterstützung von Zellenverstärkung durch Anwendung im Gewebe während Geweberegeneration nach dem Defekt eines Knorpels und/oder Knochengewebe, **dadurch gekennzeichnet** von Schritten der,
- Synthetisierung Polypropylenfumarat (PPF) Pre-polymer (101),
- Mischung PPF mit Vinylphosphonicsäure (VPA) oder Vinylphosphonicsäureester (VPES) (102),
- Nachbehandlung der Mischung durch Erwärmung sie im Anwesenheit von einem radikalen Anreger (103),
- Erhaltung von porösen oder nicht porösen Biopolymer, der verwendet wird als Gewebegerüst (104).

2. Produktionsmethode für Gewebegerüst (100) nach Anspruch 1, **dadurch gekennzeichnet, daß** es Schritte der Synthetisierung von PPF Pre-polymer (101) gibt, wo Fumarsäure und Propylenglykol beansprucht sind zur Polykondensationsreaktion in Anwesenheit von Hydrochinonradikalinhibitor und P-Toluensulfonsäurekatalysator; und da die Reaktion in mehr als Propylenglykol stattfindet, PPF Pre-polymer ist erhalten in Hydroxyl-fertiggestellter Form.

3. Produktionsmethode für Gewebegerüst (100) nach Anspruch 2, **dadurch gekennzeichnet, daß** es Schritte der Synthetisierung von PPF Pre-polymer (101) gibt, wo 0.1-0.3% nach Gewicht von Hydrochinon und 1-2% nach Gewicht von P-Toluen sulfonsäure verwendet wird.

4. Produktionsmethode für Gewebegerüst (100) nach Anspruch 3, **dadurch gekennzeichnet, daß** es der Schritt gibt der Mischung von PPF Pre-polymer mit Vinylphosphonicsäure (VPS) Komonomer (102) in Anwesenheit von 2-3% nach Gewicht von Benzoylperoxid.

5. Produktionsmethode für Gewebegerüst (100) nach Anspruch 3, **dadurch gekennzeichnet, daß** es der Schritt gibt der Mischung von PPF Pre-polymer mit Vinylphosphonicsäureester (VPES) Komonomer (102) in Anwesenheit von 2-3% nach Gewicht von Benzoylperoxid.

6. Produktionsmethode für Gewebegerüst (100) nach Ansprüche 4 oder 5, **dadurch gekennzeichnet, daß** es der Schritt gibt der Mischung von PPF mit Vinylphosphonicsäure (VPA) oder Vinylphosphonicsäureester (VPES) (102), wo die PPF Menge in der Mischung 10-90% nach Gewicht ist.

7. Produktionsmethode für Gewebegerüst (100) nach Anspruch 6, **dadurch gekennzeichnet, daß** es der Schritt gibt der Nachbehandlung von vorbereiteter Mischung (103) in einer Temperatur-kontrollierten Ofen in Anwesenheit von Benzoylperoxid in 60°C für 2 Stunden, in 85°C für 2 Stunden und in 100°C für 5 Stunden, beziehungsweise.

8. Produktionsmethode für Gewebegerüst (100) nach Anspruch 7, **dadurch gekennzeichnet, daß** nach dem Behandlungsprozess (103), der Biopolymer, die als Gewebegerüst verwendet wird, erhalten ist in der Form von einer porösen PPF-VPA Mischung oder nicht porösen PPF-VPES Mischung (104).

9. Ein poröses, biologisch abbaubares und biocides Gewebegerüst bestehend aus PPF-VPA Mischung beinhaltet 60% nach Gewicht von PPF, der produziert ist mit der Methode nach Anspruch 8.

## Revendications

1. Procédé de production d'échafaudage tissulaire (100), qui est conçu pour soutenir l'amplification cellulaire en étant appliqué au tissu pendant la régénération tissulaire après le défaut d'un cartilage et / ou d'un tissu osseux, **caractérisé par** les étapes suivantes:
- synthétiser un prépolymère de fumarate de polypropylène (PPF) (101),
- mélanger PPF avec de l'acide vinylphosphonique (VPA) ou vinylphosphonique
- ester d'acide (VPES) (102),
- durcir le mélange en le chauffant en présence d'un initiateur radicalaire (103),
- obtenir le biopolymère poreux ou non poreux qui sera utilisé comme échafaudage tissulaire (104).

2. Procédé de production d'échafaudage tissulaire (100) selon la revendication 1, **caractérisé par** l'étape de synthèse du prépolymère PPF (101) dans lequel l'acide fumarique et le propylène glycol sont soumis à une réaction de polycondensation en présence d'un inhibiteur radical hydroquinone et d'un catalyseur acide p-toluène sulfonique ; et comme la réaction a lieu en excès de propylèneglycol, le prépolymère de PPF est obtenu sous une forme à terminaison hydroxyle.

3. Procédé de production d'échafaudage tissulaire (100) selon la revendication 2, **caractérisé par** l'étape consistant à synthétiser un prépolymère de PPF (101) dans lequel 0,1-0,3% en poids d'hydroquinone et 1-2% en poids d'acide p-toluène sulfonique sont utilisés.

4. Procédé de production d'échafaudage tissulaire (100) selon la revendication 3, **caractérisé par** l'étape consistant à mélanger un prépolymère de PPF avec un comonomère d'acide vinylphosphonique (VPA) (102) en présence de 2 à 3% en poids de peroxyde de benzoyle.

5. Procédé de production d'échafaudage tissulaire (100) selon la revendication 3, **caractérisé par** l'étape consistant à mélanger un prépolymère de PPF avec un comonomère d'ester d'acide vinylphosphonique (VPES) (102) en présence de 2 à 3% en poids de peroxyde de benzoyle.

6. Procédé de fabrication d'échafaudage tissulaire (100) selon la revendication 4 ou 5, **caractérisé par** l'étape de mélange de PPF avec de l'acide vinylphosphonique (VPA) ou de l'ester vinylsulfonique (VPES) (102) dans lequel la quantité de PPF dans le mélange est 10-90 % par poids.

7. Procédé de production d'échafaudage tissulaire (100) selon la revendication 6, **caractérisé par** l'étape de durcissement du mélange préparé (103) dans un four à température contrôlée en présence de peroxyde de benzoyle à 60°C pendant 2 heures, à 85 ° C pendant 2 heures, heures et à 100°C pendant 5 heures, respectivement.

8. Procédé de production d'échafaudage tissulaire (100) selon la revendication 7, **caractérisé en ce que,** après le procédé de durcissement (103), le biopolymère à utiliser comme matrice tissulaire est obtenu sous la forme d'un mélange poreux PPF-VPA ou PPF-VPES non poreux (104).

9. Échafaudage tissulaire poreux, biodégradable et biocompatible constitué d'un mélange de PPF-VPA contenant 60% en poids de PPF, produit par le procédé selon la revendication 8.
